# EUROPEAN PATENT SPECIFICATION

(11) **EP 0 309 550 B1**
(45) Date of publication and mention of the grant of the patent: **19.06.1996**
(21) Application number: 88903675.2
(22) Date of filing: 28.03.1988
(51) Int. Cl.: C12N 15/57, C12N 15/70, C12N 15/79, C12N 9/58, C12N 1/21, C07H 15/12, C11D 7/42

(54) **NOVEL PROTEOLYTIC ENZYMES**
PROTEOLYTISCHE ENZYME
NOUVELLES ENZYMES PROTEOLYTIQUES

(30) Priority: 03.04.1987 US 35816
(43) Date of publication of application: 05.04.1989
(73) Proprietor: AMGEN INC., Thousand Oaks, CA 91320-1789 (US)
(72) Inventor: SAMAL, Babru, B., Newbury Park, CA 91320 (US); STABINSKY, Yitzhak, Boulder, CO 80303 (US)
(74) Representative: Brown, John David
(86) International application number: US8801040
(87) International publication number: WO8807581

(56) References cited:
- US-A- 3 623 957
- US-A- 3 790 482
- ABSTRACTS OF THE ANNUAL MEETING OF THE AMERICAN SOCIETY FOR MICROBIOLOGY, Atlanta, GA (US), 01-06 March 1987; B. KARAN et al., p. 150, AN H-65
- JOURNAL OF CELLULAR BIOCHEMISTRY, Suppl. 11C, UCLA Symposia on Molecular & Cellular Biology, 15 March-11 April 1987, Alan R. Liss Inc., New York, NY (US); B. SAMAL et al., p. 237, AN N-413
- GENE, vol. 34, 1985, Elsevier Science Publishers; J.A. WELLS et al., pp. 315-323
- FEBS Letters, vol. 199, no. 2, April 1986, Amsterdam (NL); JANY et al., pp. 139-144
- CHEMICAL ABSTRACTS, vol. 103, no. 1, 08 July 1985, Columbus, OH (US); JANY et al., p. 272, AN 2890j
- THE EMBO JOURNAL, vol. 3, no. 6, June 1984, Oxford (GB); PAHLER et al., pp. 1311-1314
- CHEMICAL ABSTRACTS, vol. 83, 1975, Columbus, OH (US); p. 236, AN 110435s

## Description

This invention relates to novel serine proteases isolated from a culture medium of the fungus Tritirachium album. The serine proteases of the present invention have a high degree of stability in aqueous solutions and dry detergent formulations. The invention further relates to detergent compositions containing such proteases and to the use of the proteases in detergents and cleaners or spot cleaners. In addition, the present invention further relates to DNA sequences encoding for the proteases and to a method for producing the proteases.

Serine proteases are proteolytic enzymes having a serine residue at their active site. Modification of the active site serine residue by agents such as phenylmethylsulfonylfluoride (PMSF) inactivates these enzymes. There are two classes of serine proteases, chymotrypsin-like proteases and subtilisin-like proteases. Subtilisins are serine proteases which generally act to cleave internal peptide bonds of proteins or peptides. Subtilisins are secreted by a number of Bacillus species and extensively used commercially (see U.S. patent No. 3,623,957, J. Miller, 1970, J. Appl. Bacteriol., 33, 207; Ward, O.P. 1983, pp. 251-317, Enzymes and Biotechnology, ed., W.M. Fogarty, Applied Science Publishers, London). Subtilisins have been utilized in a number of detergent formulations (see U.S. Patent Nos. 1,240,058, 3,749,671; 3,790,482; 4,266,031, U.K. Patent No. 1315937).

The use of proteases in industrial processes wnich require hydrolysis of protein has been limited due to the enzyme instability under the temperature and pH conditions associated with such processes. Although thermal inactivation of the protease may be the most important factor in restricting the industrial use of a protease, other factors such as lack of effectiveness over broad pH ranges and use of denaturing agents in detergent formulations may also have a detrimental effect regarding the use of proteases in industrial processes. The known Bacillus derived subtilisins are not ideal for all applications as detergent enzymes, in particular, application requiring greater storage stability and activity at broader ranges of pH and temperature. Therefore there is a need for a class of proteases that are characterized by high stability with respect to temperature, pH, denaturing agents and the like.

There also is a need for proteases that are compatible with detergents and have sufficient shelf-life in liquid detergent formulations to be commercially practical. Thermostable fungal serine proteases have been evaluated in detergent applications, including proteases obtained from Tritirachium album (Ebeling, W. et al., 1971, German Offenbach, 1965, 281), Malbranchea pulchella (Ong, P.S. et al., Can. J. Microbiol. 22, 165), Acremonium kiliense, Fusarium and Gibberella spp (Isono, M., et al., 1972, U.S. Patent No. 3652399). Proteinase K (EC 3, 4, 21, 14) was isolated from Tritirachium album (Ebeling, W. et al., 1974, Eur. J. Biochem. 47, 91-97) and has been extensively studied by different groups (Kraus, E. et al., 1976, Hoppe Seyler's Z. Physiol. Chem. 357, 937-947, ibid. 357, 233-237, and Morihara, K. et al., 1975, Agr. Biol. Chem. 39, 1489-1492). The three dimensional structure of proteinase K is similar to that of subtilisins (Paehler, A. et al., 1983, EMBO J. 3, 1311-1314) and there is about 35% homology of the amino acid sequence of proteinase K with that of subtilisins. (Jany, K-D., et al., FEBS Letters, 199, 139-144). Detergent compatibility of proteinase K has been suggested from its activity in the presence of high concentration of detergents (Hilz, H. et al., 1975, Eur. J., Biochem., 56, 103-108).

Proteolytic enzymes generally catalyze the cleavage of peptide bonds only within a certain range of pH and temperature. Moreover, even under optimal conditions, a proteolytic enzyme retains its activity only when its polypeptide chain is in native conformation. Unfolding of the native structure often occurs when the enzyme is exposed to extremes of pH or temperature or to certain detergent additives such as surfactants and metal-chelating agents. The latter exert their effect especially on enzymes that require metal ions such as Ca²⁺ for stabilizing their native structure, i.e., bacterial subtilisin. For proteases, partial unfolding of the native conformation of the enzyme may lead to acceleration of autodigestion and therefore, to irreversible enzyme inactiviation. Because most commercial laundry detergents have an alkaline pH, it is desirable that the enzyme utilized in such detergents be active and stable in a pH range of between 7.5 and 13 and in a temperature range of between 20-65°C. Moreover, it is desirable that the activity of such enzymes be relatively independent of calcium and magnesium ions and be compatible with surfactants and sequestrant builders. Bacterial serine proteases of the subtilisin family fulfill these requirements to some extent, however their stability in liquid detergent formulation is limited.

### SUMMARY OF THE INVENTION

The present invention relates to novel serine proteases that are characterized by improved stability in detergent formulations. In particular, the present invention describes the isolation, purification and characterization of novel serine proteases having the amino acid sequences as set forth in Figures 3 and 8 and obtained from the strain of fungus Tritirachium album Limber (ATCC 22563). The serine proteases of the present invention have been found to be superior detergent enzymes and possess a high degree of thermal stability in aqueous solutions particularly at elevated temperatures, thereby making the proteases suitable for use as additives in commercial liquid detergent formulations. The present invention further relates to the isolation and characterization of the genes encoding such proteases. The invention further relates to the use of the serine proteases of the present invention in detergents and cleaners or spot cleaners and compositions containing the serine proteases.

In addition, the present invention relates to the use of an oligonucleotide probe which hybridizes with complementary DNA sequences in the genomic or cDNA clones of the serine proteases disclosed herein. Finally the present invention relates to DNA sequences useful in securing expression in a procaryotic or eucaryotic host cell a serine protease isolated from culture media containing strain of T. album Limber (ATCC 22563) and the method for isolating such proteases.

The present invention further relates to a purified and isolated serine protease having the structural conformation (i.e., continuous sequence of amino acid residues) of a serine protease isolated from a culture of Tritirachium album Limber strain ATCC 22563 and characterized by being the product of procaryotic or eucaryotic expression of an exogenous DNA sequence.

Also, the present invention provides a process for the production of a serine protease having the structural conformation of a serine protease isolated from a culture or Tritirachium album Limber strain ATCC 22563, said process comprising growing under suitable nutrient condition procaryotic or eucaryotic host cells transformed or transfected with a DNA vector including a DNA sequence useful in securing expression in a procaryotic or eucaryotic host cell the desired serine protease and isolating desired serine protease of the expression of DNA sequences in said vector.

Also provided herein are oligonucleotide probes capable of hybridizing with a DNA sequence capable of expressing in a procaryotic or eucaryotic host cell a serine protease having the structural conformation of a serine protease isolated from culture of media of Tritirachium album Limber strain ATCC 22563.

### BRIEF DESCRIPTION OF THE DRAWINGS

Figure 1 represents the nucleotide sequence of the genomic clone for protease TW7;
Figure 2 represents the nucleotide sequence of the cDNA clone for protease TW7;
Figure 3 represents the nucleotide sequence of the coding strand, correlated with the amino acid sequence of the protease TW7;

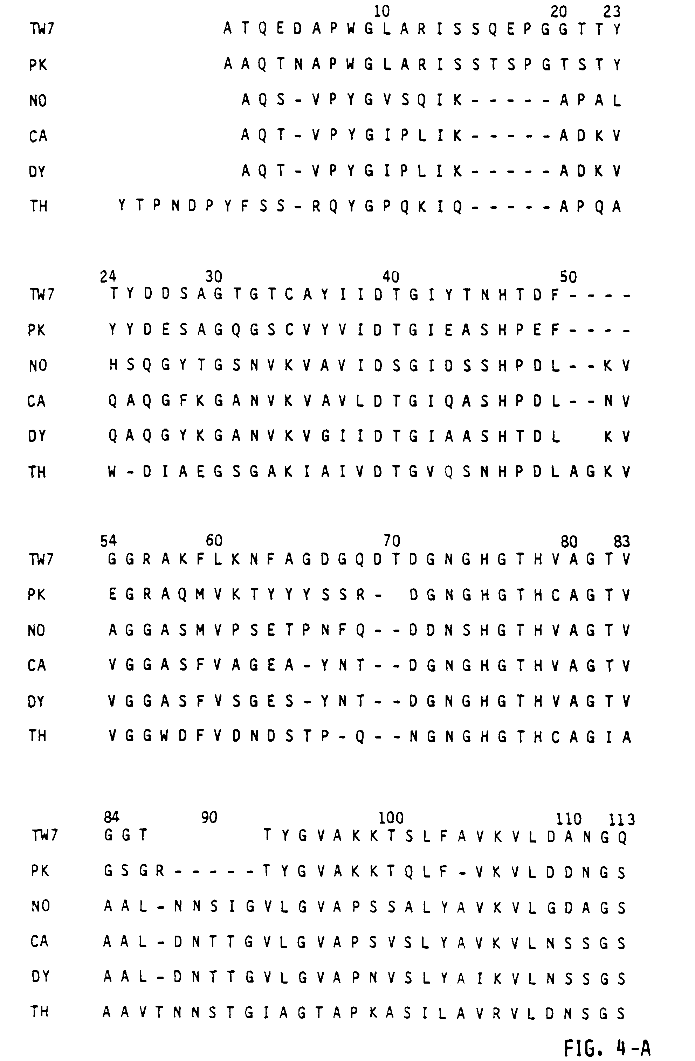

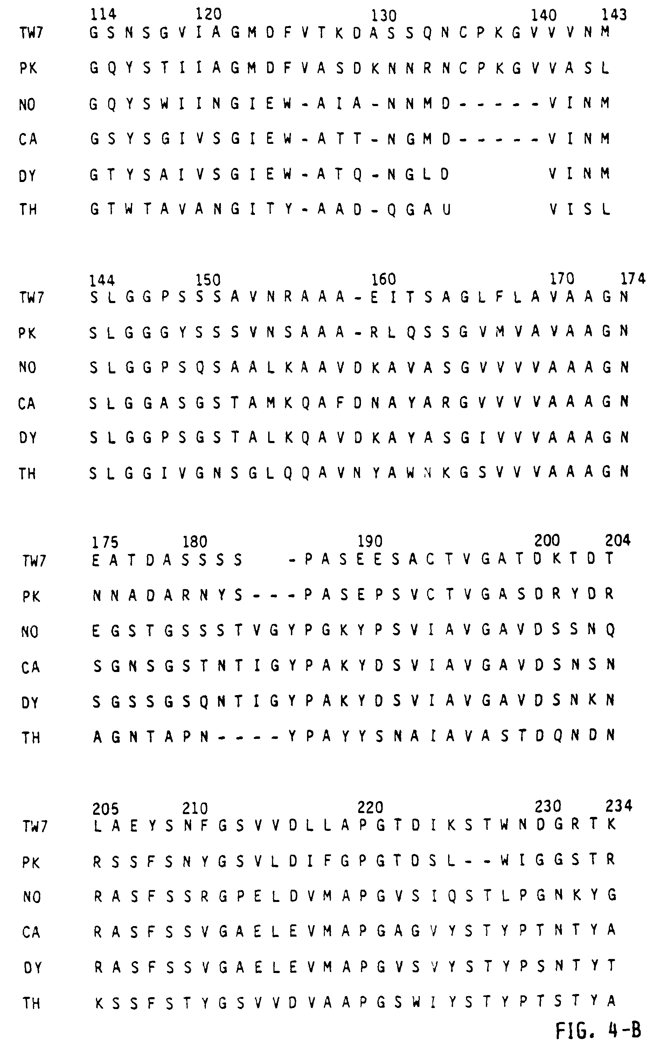

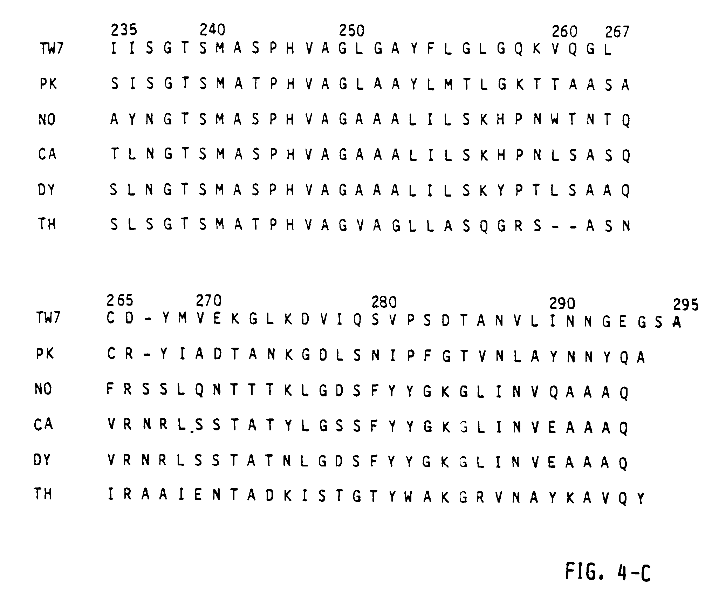

Figure 4 represents a comparison of the amino acid sequences of protease TW7 with those of proteinase K, subtilisin novo, subtilisin Carlsberg, subtilisin DY and thermitase;
Figure 5 represents the pH profiles of protease TW7;
Figure 6 represents the temperature profiles of the protease TW7;

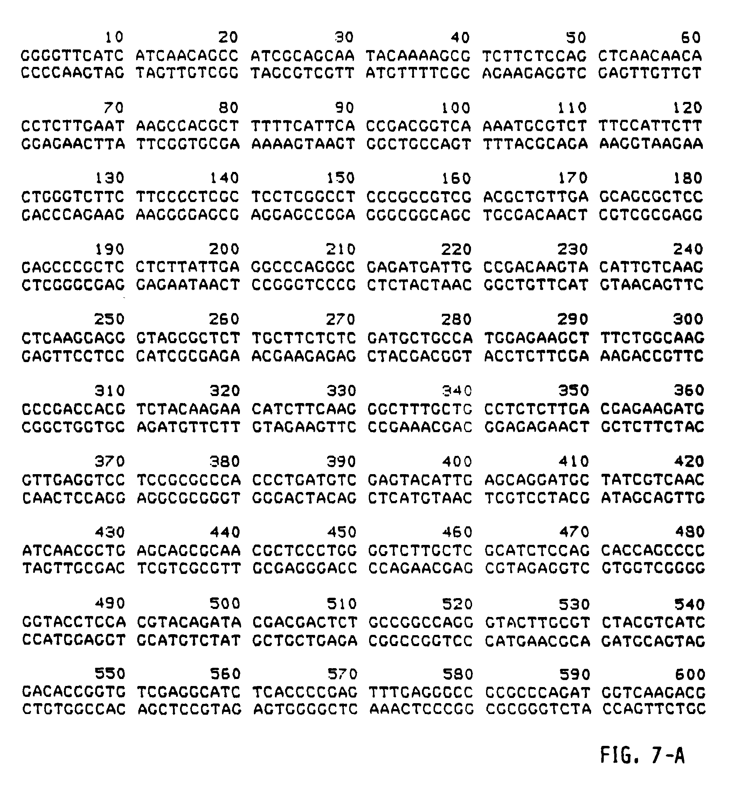

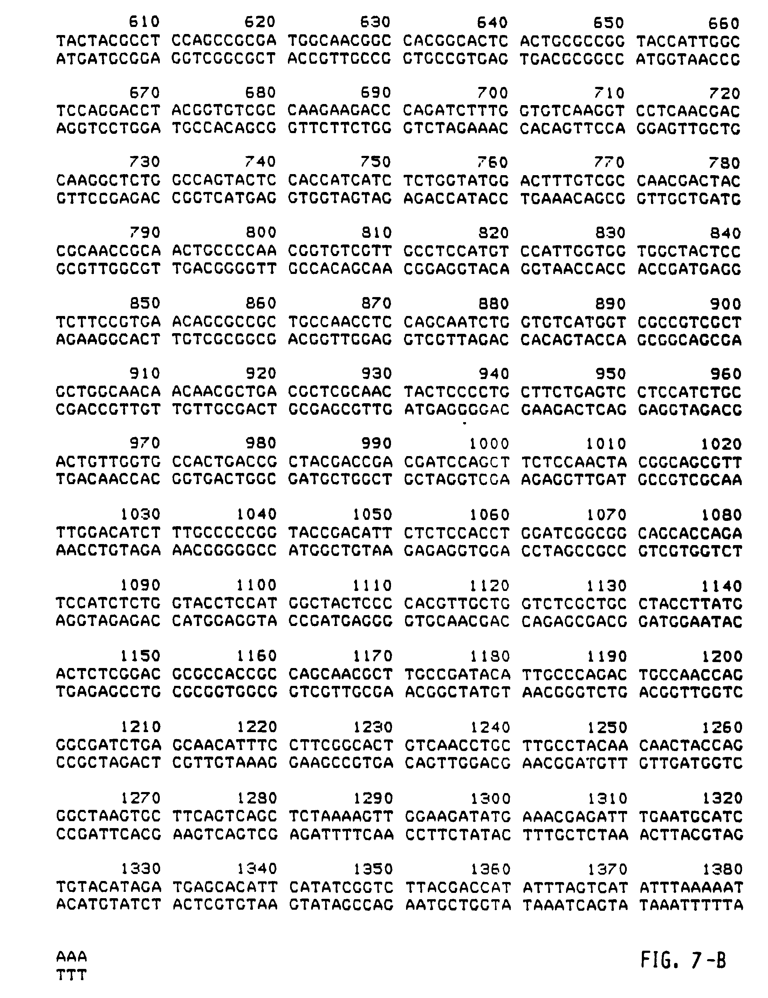

Figure 7 represents the nucleotide sequence of the cDNA clone for protease TW3;

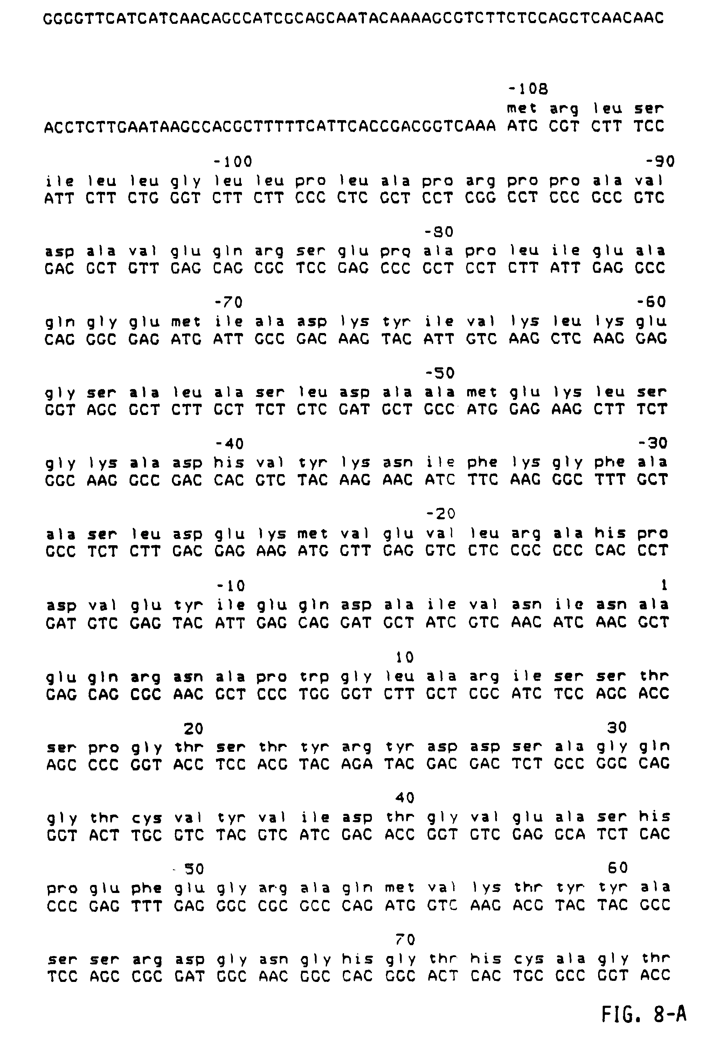

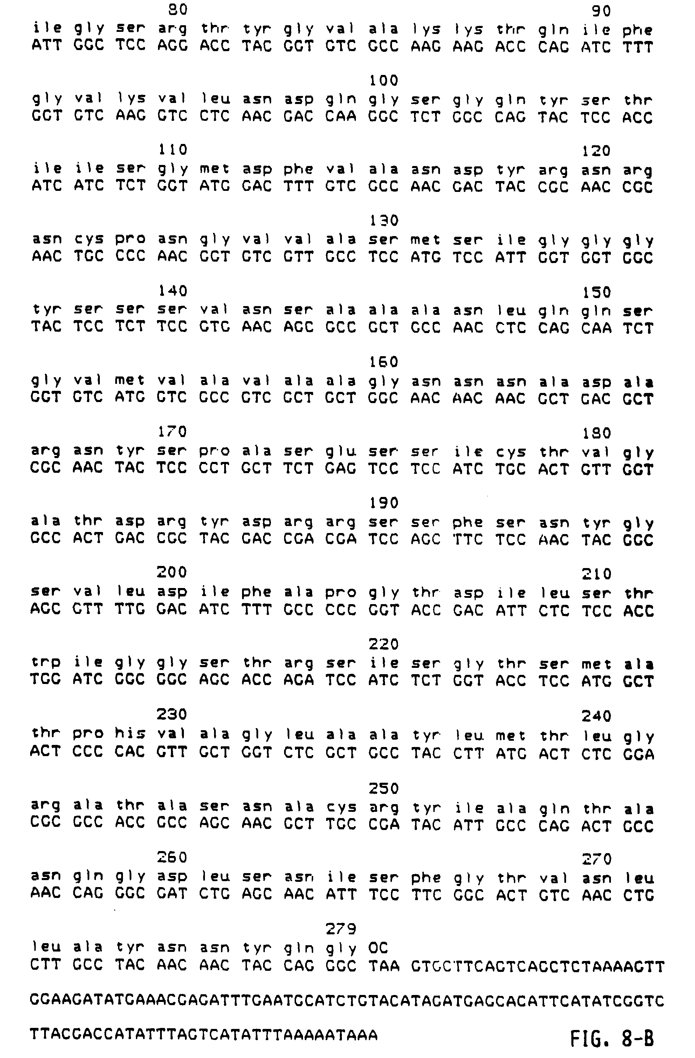

Figure 8 represents the nucleotide sequence of the coding strand, correlated with the amino acid sequence of the protease TW3;
Figure 9 represents a comparison of the amino acid sequences of protease TW3 with those of proteinase K, subtilisin novo, subtilisin Carlsberg, subtilisin DY and thermitase;
Figure 10 represents the pH profiles of protease TW3;
Figure 11 represents the temperature profiles of the protease TW3; and
Figure 12 represents a flow diagram illustrating the components utilized in construction of pCFM 1156 TW7.

### DETAILED DESCRIPTION

Because T. album Limber is a slow growing fungus capable of producing only low level quantities of proteases, production of proteinase K or any other subtilisin-like enzymes in large commercial quantities by fermenting the fungus, is not practical. In addition, the growth of the fungus T. album as well as the production of proteases are slow, making the commercialization of a subtilisin-like enzyme uneconomical.

One aspect of the present invention relates to the isolation of a gene encoding the novel serine proteases of the present invention from T. album Limber, and the cloning and expression of such a gene in a suitable microorganism. The gene was isolated using a deoxyribonucleotide oligomer that hybridizes with a gene having a DNA sequence encoding the amino acid residues around the active serine residue. Using this approach, more than one gene has been isolated from the genomic library. This suggests the production of more than one serine protease from T. album Limber. The amino acid sequences of the amino termini of the proteases were found to be different. The novel proteases, protease TW7 and protease TW3 were isolated and characterized.

The gene encoding for protease TW7 was isolated from a genomic library. In particular, the isolation of subtilisin-like genes from the genomic library of T. album comprises (1) construction of a library from the genomic DNA of T. album Limber in a pBR322 plasmid; (2) screening of the genomic library with a labeled oligonucleotide probe which hybridizes specifically with subtilisin-like genes; (3) restriction enzyme analysis of positive clones followed by Southern blot hybridization with various restriction fragments for the identification of clones carrying the entire gene; (4) subcloning of restrictive fragments carrying the entire gene in bacteriophage M13 for DNA sequence analysis; (5) designing oligonucleotide primers based on partial DNA sequence data to complete DNA sequencing of both strands of the gene.

Sequencing of both strands of the genomic gene revealed the presence of two introns. In order to express genes containing introns in microorganisms that lack splicing enzymes, e.g., B. subtilis, E. coli, etc., it is necessary to reconstruct the gene (i.e., utilizing in vitro splicing) or obtain the gene from a cDNA library. The genes encoding for protease TW7 and protease TW3 were isolated from a cDNA library. The isolation of subtilisin genes from the cDNA library of T. album Limber comprises (1) isolating of total RNA from T. album Limber; (2) fractioning of RNA on an oligonucleotide dT cellulose column and isolation of polyadenylated mRNA fraction; (3) using an oligonucleotide probe for Northern blot analysis to confirm the presence of subtilisin-like mRNA; (4) cDNA synthesis and construction of a cDNA library in a pBR322 derived plasmid; (5) screening of the cDNA library with a ³²P-labeled oligonucleotide probe that was utilized in Step 3; (6) isolation and restriction analysis of positive cDNA clones; (7) subcloning of restriction fragments from positive cDNA clones that carry the entire protein coding sequence in bacteriophage M13 for DNA sequencing; (8) using oligonucleotide primers to complete the DNA sequencing of both strands of the gene.

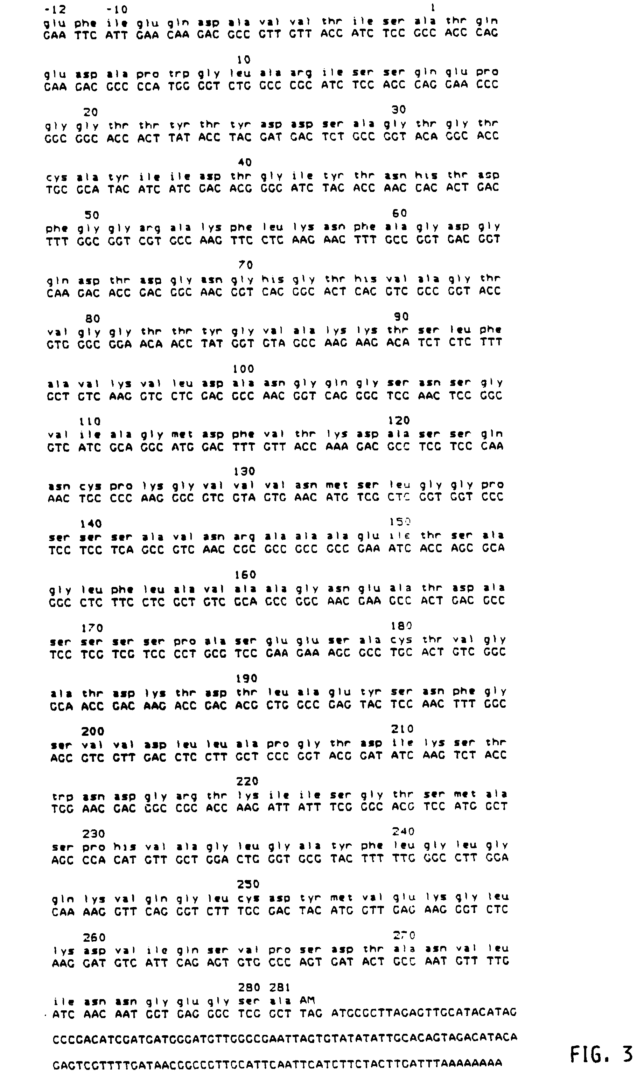

In accordance with the above procedures, two cDNA genes have been identified whose amino acid sequences as deduced from the DNA sequences indicated that their products are subtilisin-like enzymes. One of the gene products has the amino acid sequence represented in Figure 3 and was named protease TW7. Protease TW7 exhibits approximately 53% amino acid sequence homology with proteinase K from T. album Limber. The putative amino acid sequence of protease TW7 indicated that unlike proteinase K, this protease has a net negative charge and therefore may be separated from the other proteases using DEAE sepharose or DEAE-cellulose chromatography. The second of putative gene products was named protease TW3. The degree of amino acid sequence homology of this protease and proteinase K from T. album Limber was approximately 90%. The putative amino acid sequence of protease TW3 indicated that the enzyme has a net positive charge and therefore can be separated from the other proteases by CM-cellulose chromatography.

The serine proteases of the present invention were isolated from the culture broth of T. album Limber strain (CBS 348.55) ATCC Deposit No. 22563 as follows:

The particular fungus T. album strain (ATCC 22563) was grown in media containing only proteins as nitrogen sources, e.g., skim milk, bovine serum albumin or soy flour. The culture media were tested for proteolytic activity using azocasein as the substrate. When the proteolytic activity reached a plateau or started to decline, the culture broth was separated from fungal mycelia by centrifugation. Proteins in the supernatant were precipitated using ammonium sulfate. The precipitate was dissolved in 20 mM sodium phosphate buffer at pH 6.0 and the solution was dialyzed against the same buffer. The solution was passed through a column of CM-52 (Whatman) to capture proteinase K-like enzymes and then passed through a DE-52 (Whatman) column. Proteinase TW7 was eluted from the DE-52 column with 100 mM NaCl in 20 mM sodium phosphate at pH 6.0. It was dialyzed against water and then lyophilized. Protease TW3 was bound to CM-52 from which it was eluted with 200 mM NaCl in 20 mM sodium phosphate at pH 6.0.

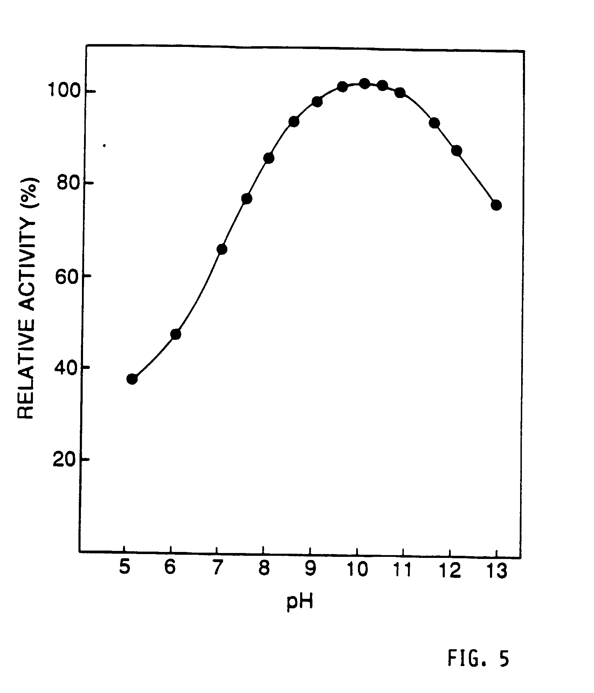

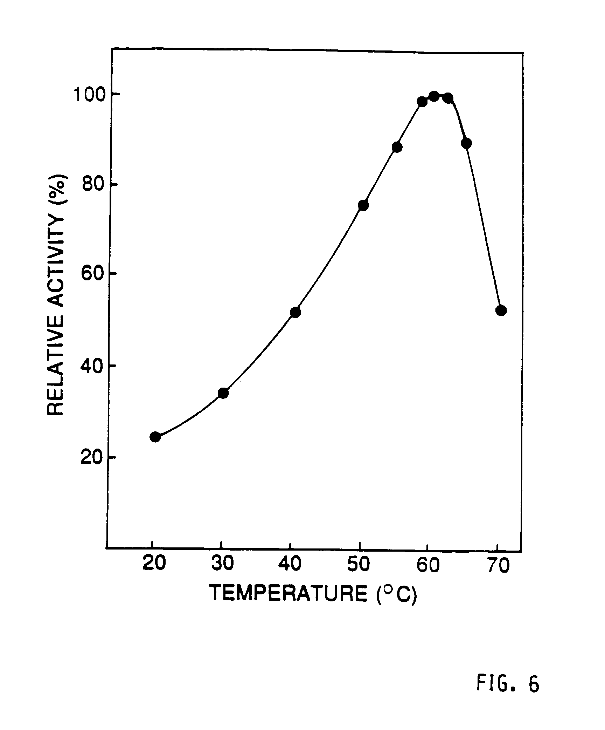

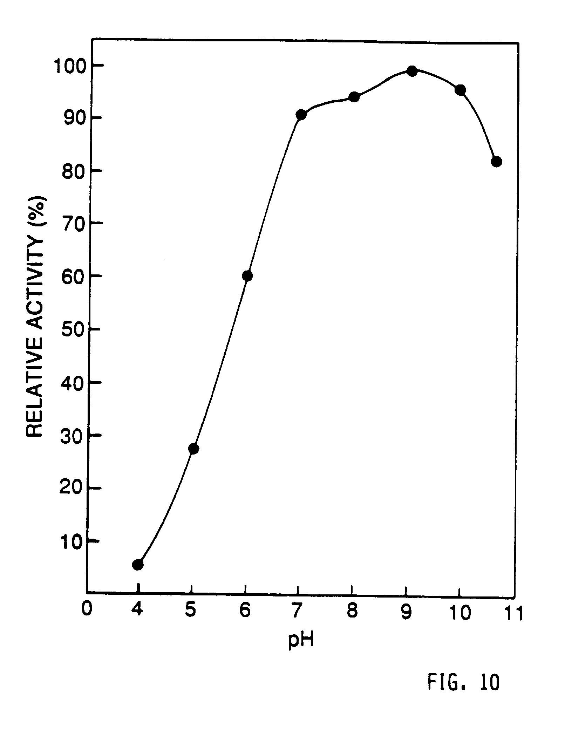

The detergent compatibility properties of the serine proteases of the present invention have been characterized. As shown in Figures 5 and 10, protease TW7 and protease TW3 are active over a wide pH range. Although the maximum activity occurs approximately at pH 10, protease TW7 has considerable activity within pH range 9 to 13. In addition, protease TW7 retains at least 45% of its maximum activity present in a buffer having pH of 6.0. As represented in Figure 6, protease TW7 has enzymatic activity over a broad temperature range. The maximum activity is between 60-65°C, although at 20°C protease TW7 retains at least 25% of its original activity. Protease TW7 is stable at 52°C in buffers of various pH values. Following incubation for a period of one hour at pH 8 and 10.3, approximately 100% of the original activity is retained, while under similar conditions a commercial subtilisin retained only up to 30% of its original activity. In the presence of 0.5% SDS at pH 8.0, protease TW7 retains 100% of its activity after one hour incubation at 52°C while the commercial subtilisin retains only about 5-6% of its original activity.

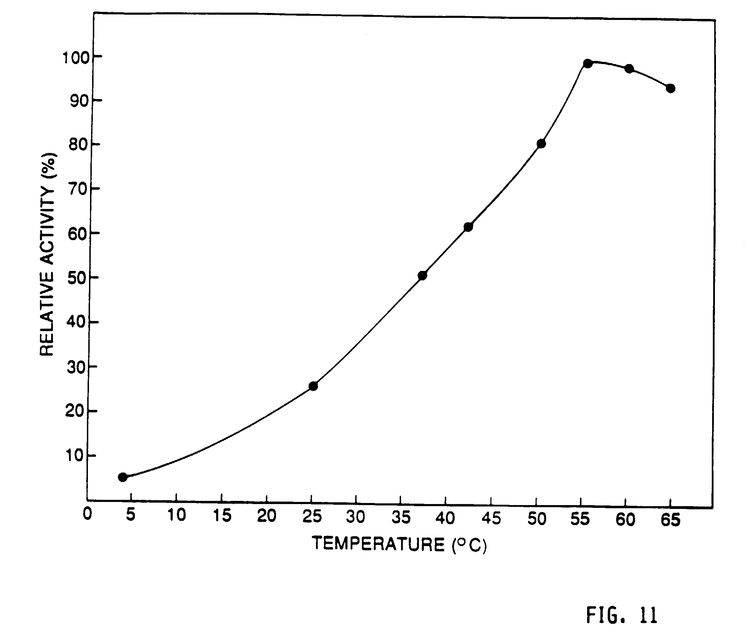

As illustrated in Figure 10, protease TW3 is also active over a broad pH range. For example, protease TW3 has its highest enzymatic activity at pH 9, although there is considerable activity within a pH range of from 7 to 10. As represented in Figure 11, the temperature range of enzymatic activity of protease TW3 is also broad. The maximum activity is between 55-65°C, although at 25°C, protease TW3 retains at least 32% of its activity. This enables protease TW3 to be active at a wide range of washing temperature. In addition, protease TW3 is very stable at 50°C in buffers of various pH values. Following one hour incubation at pH 4.0, 8 and 10.0, 50-90% of the original activity of protease TW3 is retained, while under similar conditions a commercial subtilisin retains only minimal activity.

The stability of the serine proteases of the present invention have been evaluated in different laundry detergent formulations. Any endogenous enzyme that was present was inactivated prior to evaluation by heating the detergent formulation at 65°C for one hour.

In addition to the enzyme the commercial washing powder composition of the present invention will generally contain:
(a) At least one surfactant which may be anionic, non-ionic, or amphoteric, or a water-soluble soap. Typically, an anionic surfactant (e.g. a linear alkyl aryl sulphonate) is used in admixture with a non-ionic (e.g. an alkyl phenyl polyglycol ether) in amounts of 5-30 and 1-5 percent by weight, respectively, of the washing composition.
(b) One or more builders, preferably having a concomitant sequestering function. Sodium tripolyphosphate, sodium citrate, sodium silicate, and zeolites are examples of such compounds, usually constituting from 10 to 70 percent by weight of the detergent composition.
(c) A bleaching agent, preferably a peroxy compound such as sodium perborate, typically incorporated in an amount up to 30 percent by weight of the composition.
(d) Ancillary agents, such as carboxymethyl cellulose, optical brighteners and perfumes. If required, a pH-adjusting agent is added to give a pH of the laundering medium in the range of from 8.0 to 10.5.

The particulate protease preparation of the invention is added in an amount calculated to give a protease activity of at least 0.1 Anson units (AU, vide infra), preferably 0.5-2.5 AU per 100 g of washing composition. If required, balance to 100 percent may be established with an inorganic filler, preferably sodium sulphate.

Liquid detergent compositions may be prepared from enzyme slurries, preferably in non-aqueous media. Typically, such slurries may consist of a suspension of finely ground protease concentrate in a liquid non-ionic surfactant, for example Tergitol 15 S 9 or a mixture of such surfactants. Usually, the slurry will also contain one or more inorganic fillers, such as finely ground sodium chloride, optionally in admixture with a suspension stabilizer, for example fumed silica (Aerosil 200). Tergitol and Aerosil are trade marks.

The protease slurry of the invention is added in an amount calculated to give a protease activity of at least 0.1 AU preferably 0.5-2.5 AU per 100 g of liquid detergent composition.

The washing compositions may be prepared in the usual manner, for example by mixing together the components. Alternatively, a pre-mix is made, which is then mixed with the remaining ingredients.

The following Examples will further serve to illustrate the invention although it will be understood that the invention is not limited to these specific examples.

Because of the good stability and activity properties described, the proteolytic enzyme according to the invention can be used in all fields where proteolytic enzymes are generally used. In particular, it can be used for detergents and cleansers or spot removers, as a depilatory in tanning, and also in the food industry for the preparation of protein hydrolysates and in serology for the detection of incomplete antibodies. It is particularly advantageous for use in the food industry and in serology that the enzyme according to the invention has such an excellent stability in the solid or dissolved form that physiologically acceptable quantities of calcium ions may not be necessary to stabilize the enzyme in aqueous solutions, in contrast to those of other enzyme preparations.

### Example 1

### Production of serine protease from Tritirachium album Limber.

The fungus, Tritirachium album Limber (CBS348.55, ATCC 22563) was obtained from American Type Culture Collection, 912301 Parklawn Dr., Rockville, MD. The fungus belongs to the family Moniliaceae (Limber, 1940, Mycologia, 32,23-30). Typical features of the fungus include hyaline mycelium, sparingly branched hyaline conidia, pure white dense mycelliar mat forming low dome or hemisphere. According to the Centralbureau voor Schimmelcultures (Oosterstraat 1, Baarn, P.O. Box 273, 3740 Ag Baarn, Netherland, ref. MAAS/tvs/714, dated October 4, 1985) the strain was originally isolated from deceased skin. This strain is not the same strain (CBS 747.69) from which proteinase K was isolated. Tritirachium album was propagated on malt-peptone-agar plates that contained 3% malt extract, 0.3% casein peptone and 2% bactoagar. The fungus was allowed to sporulate at room temperature for 7-8 days. Spores were collected in 15-20 ml of sterile distilled water and were maintained at room temperature for at least 30 min. before inoculation into a liquid media.

The composition of the liquid media for the production of proteases was either 2% skim milk and 0.17% yeast nitrogen base (Cat. No. 0335-15, obtained from Difco Laboratories, Detroit, MI) or 1% bovine serum albumin (BSA), 1% glucose and 0.17% yeast nitrogen base. The BSA containing mediam was sterilized by passing through a 0.45µ filter while the skim milk containing media was autoclaved for 30 minutes. 500 ml of each media was inoculated with the spore suspension and the resulting mixture was shaken. Four to five drops of antifoam were used to suppress foaming during shaking. The production of extramycelial proteases was monitored using either N-succinyl-alanyl-alanyl-prolyl-phenylalanine nitroanilide (Delmar et al., Anal Biochem. 99, 316) or azocasein (Charney, J. et al. 1947. J. Biol. Chem. 177, 501) as substrate. It was found that the production of protease was media-dependent. In skim milk media, the largest amount of protease was produced within 8-9 days of culture, while in BSA media the maximum production of protease did not occur until after 14-15 days of culture. In both media, protease activity declined after achieving maximum production level.

### Example 2

### Design and synthesis of a probe for detection of subtilisin-like genes.

Subtilisin-like enzymes share a high degree of sequence homology around their active site serine residue. Serine at position 221 has previously been identified as the reactive serine in serine proteases. Moreover, the assignment of a serine protease to the subtilisin family is based on the amino acid sequence GLY-THR-SER-MET-ALA Amino acid sequence alignment of several subtilisin-like enzymes revealed that the homology stretches beyond this sequence as follows: 
                    SER                              LEU
GLY-THR-SER-MET-ALA-   -PHE-HIS-VAL-ALA-GLY-   -ALA-ALA
                    THR                              ALA
 (Stahl, M.L. et al., 1984, supra; Wells, J.A. et al., 1983, supra; Vasantha, N. et al., 1984, supra; Svendsen, I. et al., 1986, FEBS Letters 196, 228-232; Koide, Y. et al., 1986, J. Bacteriol. 167, 110-116; Kaneda, M. et al., 1984; J. Biochem. 95, 825-825; Jany, K.-D. et al., 1985, Biol. Chem. Hoppe Seyler 366, 485-492). Analysis of the DNA sequences encoding the amino acids in subtilisins also showed high degree of conservation. Based on these observations, the following deoxyoligonucleotide (41-mer) was designed for probing genes that contain similar coding sequences: 5' GCT GCT AIT CCG GCA ACG TGA GGA GTC GCC ATG GAC GTT CC 3' The probe was synthesized using the phosphotriester method of Beaucage et al. (1981, Tetrahedron Letters 22, 1859-1862).

This oligonucleotide probe hybridizes with complementary DNA sequences in the genomic clone or cDNA clone of the serine protease at a temperature range of from 50-78°C depending on the number of mismatches. Therefore, when using this probe, it is necessary to include hybridization and washing at several temperatures and salt concentrations for adequate stringency. The 41-mer oligonucleotide may be used for probing genes encoding for subtilisin-like enzymes in genomic and cDNA libraries as well as mRNA encoding subtilisin-like enzymes.

### Example 3

### Construction of the genomic library and isolation of the gene for protease TW7.

Mycelia were collected on miracloth, as previously described, following 9-15 days of fungal growth. Mycelia were weighed and quickly frozen in dry ice and isopropanol. To 10 g of frozen mycelia was added 10 g of autoclaved alumina and 20 ml of lysis buffer (4% SDS, 20 mM Tris-HCl, pH 7.5, 10 mM EDTA, 0.15M NaCl). The resulting mixture was ground for 5 minutes in a sterile mortar using a pestle. An additional 20 ml of buffer and 40 ml of phenol-chloroform-isoamyl alcohol (25:24:1) was added to the ground mixture and the lysate was shaken for 20-30 min at room temperature and centrifuged for 10 min. The aqueous phase was extracted with phenol and chloroform. Ribonuclease A and proteinase K were added to the lysate to eliminate RNA and protein respectively from the DNA preparation. Ethanol was added to the lysate and DNA was spooled, suspended in TE (20 mM Tris-HCl, pH 8.0, 1 mM EDTA) and stored at 4°C.

A complete digestion of the genomic DNA isolated from T. album was conducted using restriction enzymes EcoRI and BamHI to construct a library in plasmid vector pBR322. The vector pBR322 was also digested with the same enzymes and then treated with alkaline phosphatase. Following ligation of the T. album DNA fragments with the pBR322 vector, transformation of competent HB101 cells was conducted in accordance with the procedures of Mandel et al., 1970, J. Mol. Biol. 53, 159-162. Ampicillin-resistant colonies were probed for the presence of subtilisin-like genes using the 41-mer oligonucleotide probe of Example 2. Colony lift and hybridization was conducted according to the procedures of Grunstein et al., 1975, Proc. Natl. Acad. Sci. USA, 72, 3961-3965 except that a gene screen membrane (NEF-972, New England Nuclear) was used instead of nitrocellulose. Filters were processed for DNA denaturation in situ, followed by neutralization, drying and baking at 80°C in vacuum. Prehybridization was conducted at 55°C for 3-4 hours in 5X Denhardt's solution containing 200 µg tRNA/ml. Hybridization was carried out at 55°C for 20 hours in 5X SSC, 1% SDS, 1X Denhardt's solution and 200 µg tRNA/ml. The filters were washed in stringent conditions, 2X SSC at 55°C until background radioactivity was negligable. A second round of screening of putative positive colonies with the 41-mer oligonucleotide probe was conducted by reinoculating the putative positive clones onto L agar plates containing 50 µg ampicillin/ml and repeating the screening steps described above. Following the second round of screening, only one positive clone was obtained. The positive colony was cultured in Luria broth containing 50 µg ampicillin/ml. From the overnight culture, a plasmid containing the gene for protease TW7 was isolated using the procedures described by Birnboim, Methods in Enzymol. 100, 243-154, (1983). For purposes of Southern blotting and extensive restriction mapping, the plasmid was purified using cesium chloride ethidium bromide gradients and ultracentrifugation. A 2.8 kb fragment of T. album DNA was found to contain the gene for protease TW7.

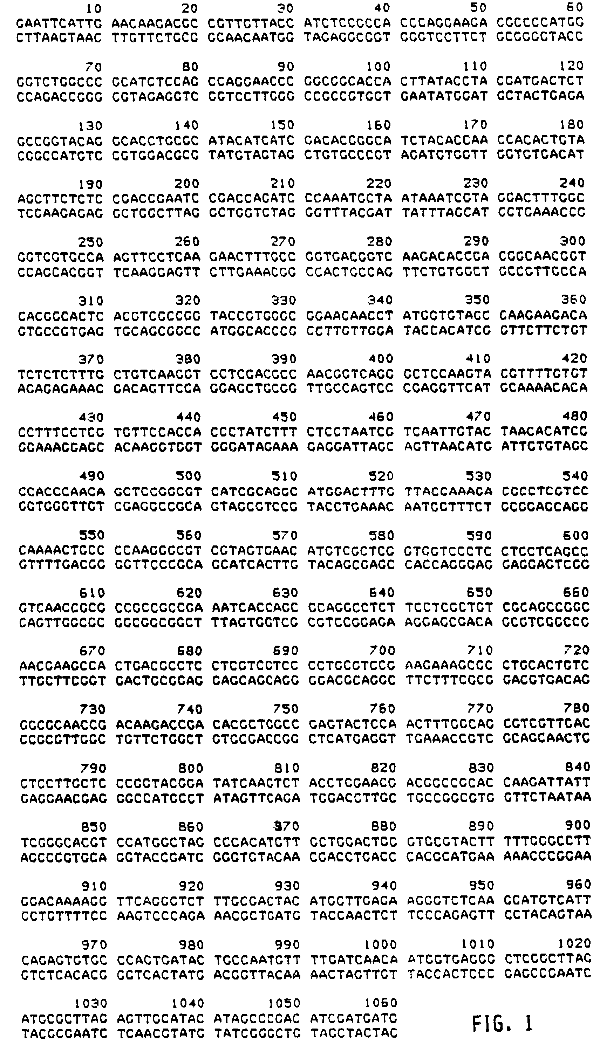

Restriction fragments of this plasmid generated by different enzymes were resolved on agarose gels by electrophoresis, transferred onto gene screen plus® membranes then probed with the 41-mer oligonucleotide probe of Example 2 to identify the fragment containing the complete gene for protease TW7. From this, it was determined that an EcoRI-ClaI fragment containing 1056 nucleotides contained the gene. This fragment was then subcloned in the bacteriophage M13mp18 and M13mp19 for single strand DNA sequencing using the universal primer (Messings, J. 1983; Methods in Enzymol. 101C, 20-75). The dideoxy chain termination procedure described by Sanger, F. et al., (1977), Proc. National Acad. Sci. USA, 74, 5463 was used for DNA sequencing. Once some partial DNA sequence was obtained, additional oligonucleotide primers were used to complete the sequencing of the two strands of the gene. The nucleotide sequence of the gene thus characterized is represented in Figure 1. The gene contains 1056 nucleotides and is defined by a site for restriction enzyme EcoRI on the 5' end and a ClaI on the 3' end. Restriction mapping of the fragment reveals the presence of unique sites for the restriction enzymes HindIII, KpnI and BglI. The putative protease encoded by this gene was named protease TW7.

The isolated gene for protease TW7 encoded the complete amino acid sequence of the mature protein and 12 amino acids of a putative "pro" region. The gene encoding the mature protease TW7 is interrupted by two introns. These introns are of 54 and 84 nucleotides in length. The exact position of the introns were determined by comparing the nucleotide sequences of the genomic DNA with that of the cDNA for protease TW7. The two introns begin with the nucleotide sequence GT and end with the sequence AG. TAG is used as the termination codon which is followed by another TAG at an 8 codon interval. The putative processing of mRNA occurs between sequences coding for serine (the last amino acid of the possible pre-pro sequence) and alanine (the first amino acid) of the mature protease TW7.

### Example 4

### Construction of the cDNA library and isolation of the cDNA gene for protease TW7.

Although the complete gene encoding the mature protease TW7 was obtained from the genomic library, this gene cannot be used for the expression of the proteolytic enzyme in microorganisms such as B. subtilis and E. coli because these organisms lack the enzymes for removing the intron sequences from the nascent RNA to form the functional messenger RNA. The isolation of functional messenger RNA from the T. album Limber fungus was attempted to obtain a complementary DNA gene, which may be expressed in microorganisms suitable for the production of protease TW7.

T. album Limber was grown in the skim milk media described in Example 1 for 9 days, after which mycelia were collected on one layer of miracloth. To about 20 g of freshly harvested mycelia was added 100 g of sterile alumina, 45 ml of lysis buffer (4% SDS, 1 mM EDTA, 100 mM Na acetate, pH 5.0) and 20 ml of phenol:chloroform:isoamyl alcohol (25:24:1), then mycelia were ground in a sterile mortar with a pestle for 20 min. After addition of 50 ml of buffer and 50 ml of phenol:chloroform: isoamylalcohol, the lysate was shaken at room temperature for 30 minutes before centrifuging at 5000 x g for 15 minutes. Following phenol:chloroform extraction, 0.1 volume of 3M ammonium acetate and 2.5 volumes of ethanol was added and the nucleic acids were allowed to precipitate at -20°C overnight. The polyadenylated RNA species was isolated using oligo dT-cellulose in accordance with the procedures described by Maniatis et al. (1982, supra). In order to prevent RNA breakdown during the manipulation of samples, all buffers were treated with 0.1% diethyl pyrocarbonate and then autoclaved for 60 minutes. The isolated mRNA population was precipitated with 2.5 volumes of ethanol following the addition 0.1 volumes of 3M ammonium acetate.

Northern blot analysis of the mRNA population was conducted following the glyoxal-DMSO procedure described by Maniatis et al. (1982). The mRNA species were separated on a 1.1% agarose gel, blotted onto a gene screen plus membrane (NEF-976, New England Nuclear) and hybridized with a kinased oligomer probe representing the amino terminus of protease TW7 and having the nucleotide sequence: 5' TGGGGCGTCTTCCTGGGTGGC 3' The prehybridization and hybridization were carried out at 55°C followed by stringent washing at 55°C and 60°C. Upon exposure of the filter to x-ray film, a single mRNA species of about 2000 nucleotide bases long was identified in the mRNA population to contain the sequence coding for protease TW7.

Double stranded cDNA was synthesized on polyA+ mRNA template following the procedures described by Okayama et al., (1982), Mol. Cell Biol., 2, 161-170. Competent E. coli HB101 cells were transformed with the plasmid vectors containing the cDNA inserts (Hanahan, 1983, J. Mol. Biol. 166, 557-580). Transformed colonies on nitrocellulose filters were replica plated onto a second set of nitrocellulose filters. The master and the replica filters were incubated at 37°C on L agar plates containing 50 µg ampicillin/ml, until the colonies grew up (generally 2-3 hours for master plates and 5-6 hours for replica). Master filters were stored at 4°C while the replica filters were incubated overnight on L agar plates containing 100 µg chloramphenicol/ml for plasmid amplification. Replica filters were processed for DNA denaturation, renaturation, baking, prehybridization followed by hybridization with the oligomeric probe as used for the mRNA detection for Northern blot. Following a second series of screening in which the isolated single positive colonies were identified, plasmid DNA was prepared in accordance with the procedures of Birnboim, (1983, supra). Nucleotide sequencing of the positive clones was conducted essentially as described for the genomic clone in accordance with Example 3.

### Example 5

### Characterization of the cDNA gene for protease TW7.

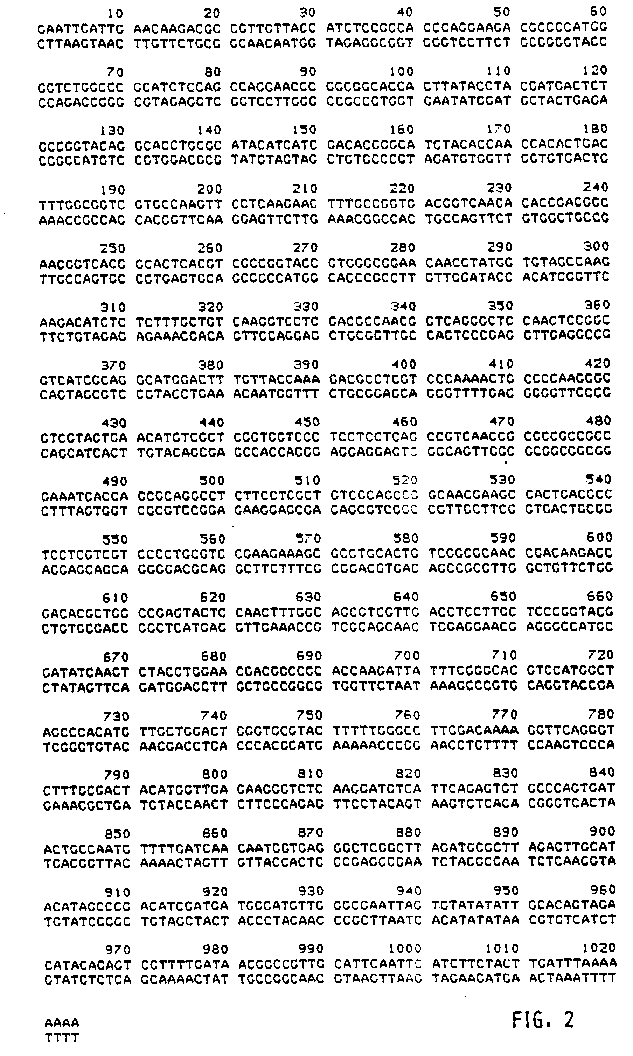

The nucleotide sequence of the cDNA clone has been determined from single stranded and double stranded DNA. Figure 2 represents the nucleotide sequence of the cDNA for protease TW7. The sequence is 1016 bases in length and ends with a polyA tail. The restriction map of the clone reveals the presence of an EcoRI site proximal to the 5' end of the sequence coding for the mature, secreted form of the gene product.

The nucleotide sequence of the cDNA clone for the protease TW7 gene is identical to the genomic clone, except for the lack of introns in the cDNA clone.

### Example 6

### Construction of the cDNA library and isolation of the cDNA gene for protease TW3.

The isolation of functional messenger RNA from the T. album fungus was attempted to obtain a complementary DNA gene which could be eventually expressed in industrial microorganisms for large scale production of protease TW3.

T. album was grown in a BSA media for 15 days after which mycelia were collected on one layer of miracloth. To about 20 g of freshly harvested mycelia 100 g of sterile alumina and 45 ml of lysis buffer (4% SDS, 1 mM EDTA, 100 mM Na acetate, pH 5.0), 20 ml of phenol:chloroform:isoamyl alcohol (25:24:1) were added then mycelia were ground in a sterile mortar with a pestle for 20 min. Following addition of 50 ml of buffer, and 50 ml of phenol:chloroform: isoamylalcohol, the lysate was shaken at room temperature for 30 minutes before centrifuging at 5000 x g for 15 minutes. Following extraction with phenol:chloroform, 0.1 volume of 3M ammonium acetate and 2.5 volumes of ethanol were added and the nucleic acids were allowed to precipitate at -20°C overnight.

The isolation of polyadenylated RNA species was carried out using oligo dT-cellulose in accordance with the procedures described by Maniatis et al. (1982, supra). The isolated mRNA population was precipitated with 2.5 volumes of ethanol following the addition of 0.1 volumes of 3M ammonium acetate.

Northern blot analysis of the mRNA population was conducted following the glyoxal-DMSO procedure described by Maniatis et al. (1982). mRNA species were separated on a 1.1% agarose gel, blotted onto a gene screen plus® membrane (NEN-976, New England Nuclear) and hybridized with kinased oligomer probes, prepared in Example 2. The prehybridization and hybridization were conducted at 55°C followed by stringent washing at 55°C and 60°C. Upon exposure of the filter to x-ray film, a single mRNA of about 2000 nucleotide bases long was identified in the mRNA population to contain the sequence coding for protease TW3.

In order to prevent RNA breakdown all buffers used in these examples were treated with 0.1% diethyl pyrocarbonate and autoclaved for 60 minutes.
Double stranded cDNA was synthesized on polyA+ mRNA template following the method of Okayama et al. (1982) Mol. Cell Biol., 2, 161-170. Competent E. coli HB101 cells were transformed with the plasmid vectors containing the cDNA inserts, (Hanahan, 1983, J. Mol. Biol. 166, 557-580). Transformed colonies on nitrocellulose filters were replica plated onto a second set of nitrocellulose filters. The master and the replica filters were incubated at 37°C on L agar plates containing 50 µg ampicillin/ml, until the colonies grew up (generally 2-3 hours for master plates and 5-6 hours for replica). Master filters were stored at 4°C while the replica filters were incubated overnight on L agar plates containing 100 µg chloramphenicol/ml for plasmid amplification.

Replica filters were processed for DNA denaturation, renaturation, baking, prehybridization followed by hybridization with the oligomeric probe used for the mRNA detection for Northern blot. After a second series of screening in which the isolated single positive colonies were identified, plasmid DNA was prepared (Birnboim, 1983, Methods in Enzymol. 101C, 20-75). Nucleotide sequencing of the positive clones was carried out as follows: A complete restriction analysis of the clone was followed by subcloning different fragments into M13mp18 and mp19 for single strand DNA sequencing. Double strand sequencing was carried out by the use of sequence-specific primers. The dideoxy chain termination procedure (Sanger, F. et al., 1977, Proc. Natl. Acad. Science USA, 74: 5463) was used for DNA sequencing.

### Example 7

### Characterization of the cDNA gene for protease TW3.

A full length cDNA clone was obtained for the protease TW3. The clone codes for the mature protease as well as the putative prepro region of the protease. There are four ATG in the open reading frame preceding the sequence coding for the mature protein. Most probably the first ATG codes for the initial methionine as it is followed by an area enriched in hydrophobic amino acids (Von Heijne, G. 1986, Nucleic Acids Res. 14, 4683-4690). The putative pro region is comprised of about 100 amino acids, a situation very similar to in subtilisin (Stahl et al., 1984 supra).

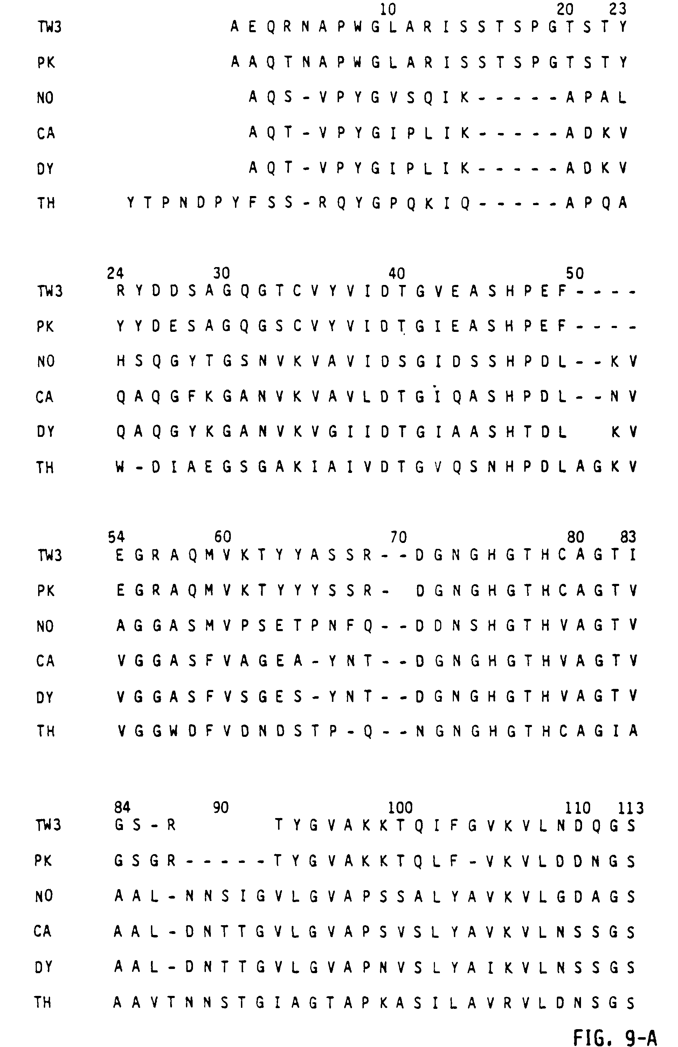

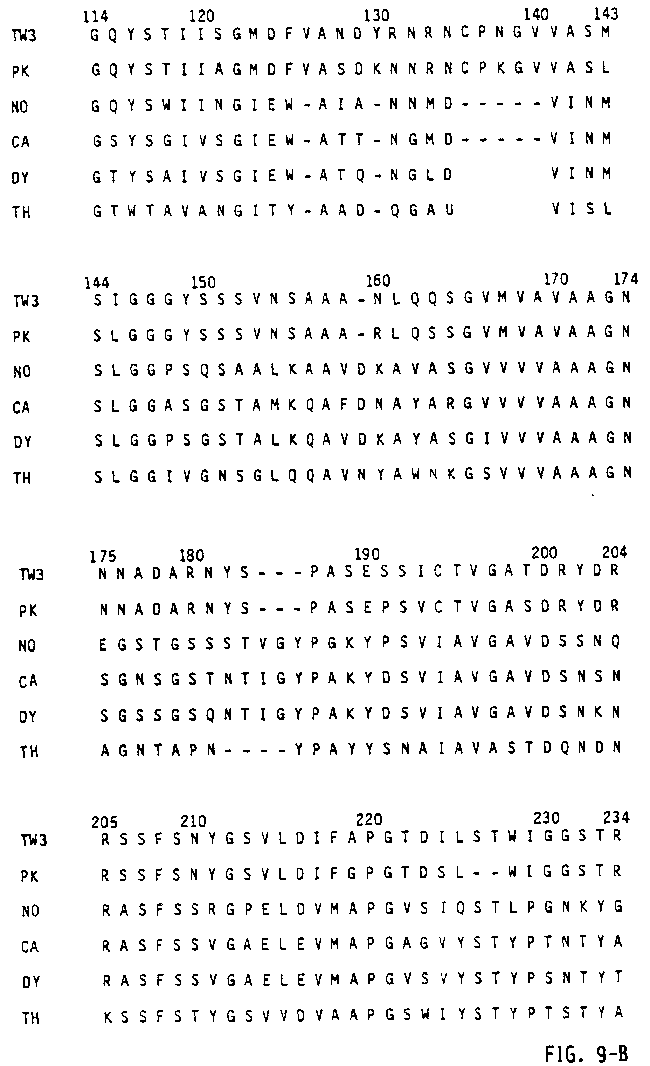

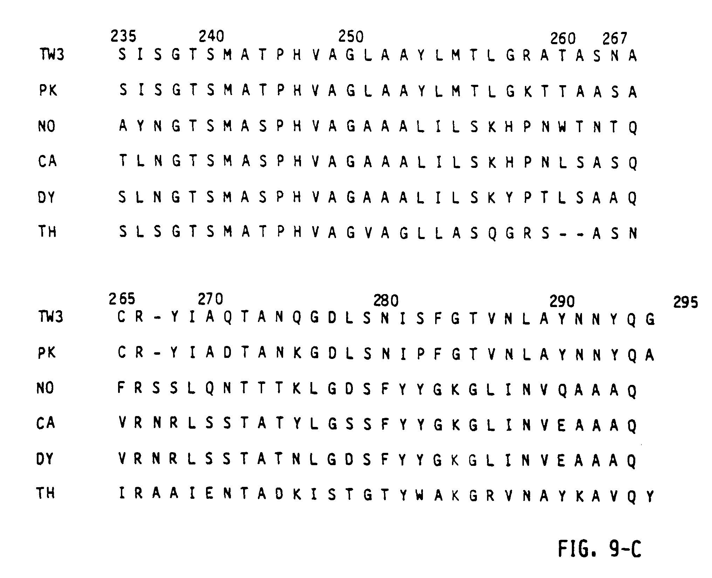

The amino acid sequence of the mature protein as determined from the nucleotide sequence has a large percentage of homology with that of proteinase K. There is approximately 90% homology between these two proteases. There are certain positions where the amino acid resembles that in subtilisins, but not to proteinase K. For example, at positions 143, a methionine residue occurs in all subtilisins as well as in protease TW3, while a leucine residue is present at that position in proteinase K. Similarly at position 219, an alanine residue is present in protease TW3 and subtilisins, but not in proteinase K. In addition, the amino acid fragment, Ser-Thr-, is absent from proteinase K while being present in all others in Figure 9 at position 226 and 227.

### Example 8

### Determination of proteolytic activity.

The proteolytic activity of serine proteases in the following Examples was determined using azoalbumin as a substrate. Aliquots (20 µl) of enzyme solution or enzyme buffer (for controls) were mixed with 1 ml of 0.6% azoalbumin in 0.05M Tris-HCl pH 8.2 (unless otherwise mentioned) and the hydrolytic reaction was conducted at room temperature. The reaction was terminated after 20 min. upon addition of 10% trichloracetic acid (400 µl). The hydrolysate was separated from the precipitated protein by centrifugation and its optical density at 410 nm, as compared to the control was measured.

Protease activity was also assayed using azocasein as the substrate. To a final volume of 500 µl, 20 µl of azocasein (5% solution in 0.2M Tris-HCl, pH 7.5, 1mM CaCl₂), 20 µl of enzyme (1-10 µg) and 460 µl of 50 mM Tris-HCl, pH 7.5 were added. The samples were incubated at 37°C for 30 min. Following incubation, 500 µl of 10% TCA was added to the samples and the samples were incubated on ice for 15 min. After centrifugation for 2 min. 800 µl of supernatant was added to a tube containing 200 µl of 1.8N NaOH. The optical density of the sample was then measured at 420 nm against the control.

### Example 9

### Isolation and purification of protease TW7 from the culture broth of Tritirachium album Limber.

Protease TW7 was separated and purified from the extracellular media of the T. album culture. A 0.5 liter aliquot of the T. album culture broth obtained after 15 days of fermentation in the BSA-glucose media described in Example 1 was centrifuged for 10 min. Proteins in the clear supernatant were precipitated using ammonium sulfate (180 g) and collected by centrifugation. The precipitate was suspended in 0.02 M sodium phosphate, pH 6.0 (50 ml) and insoluble material was removed by centrifugation. The proteins in the supernatant were reprecipitated with acetone (2.5 volumes). The precipitate was collected by centrifugation and collected on a sintered glass funnel. The precipitate was then dissolved in water (40 ml) and the resulting solution was dialyzed at 4°C against 0.02 M sodium phosphate at pH 6.0. The dialyzed solution was cleared by centrifugation and then passed through a column (2.5 x 10 cm) of carboxylmethyl-cellulose (CM-52, Whatman) at a rate of 2 ml per minute, followed by washing the column with 0.02M sodium phosphate pH 6.0 (30 ml). The flow through and washing solutions were combined and the proteins were precipitated by adding 2.5 volumes of acetone. The precipitate was separated by centrifugation, filtered, and dried under vacuum. The acetone powder (185 mg) was dissolved in 0.02 M sodium acetate pH 5.0 (2 ml) and loaded onto a Sephadex G-75 molecular sieve column (2.5 x 90 cm). Fractionation on the molecular sieve column was conducted with 0.02 M sodium acetate pH 5.0 at a flow rate of 6 ml per hour. Fractions of 2 ml were collected and monitored for u.v. absorbance at 275 nm. Of the three major peaks that were eluted from the column, only one showed proteolytic activity in hydrolyzing the chromogenic protein-substrate azoalbumin. The enzyme in this peak (between 102-110 ml) was precipitated with acetone (2.5 volumes) and collected by centrifugation. The precipitate was dissolved in 2 mM calcium acetate (20 ml) and the solution was dialyzed at 4°C against water, and then lyophilized. SDS-PAGE of reduced sample demonstrated protease TW7 as one major band (>95%) with an apparent molecular weight of 35,000 daltons.

### Example 10

### Amino terminus analysis of protease TW7.

Protease TW7 was purified from the culture supernatant as described in Example 9. The purified protein was then inactivated with PMSF (phenylmethylsulfonylfluoride) and further purified using HPLC. The amino terminus was determined by reconstituting the protein in 50% trifluoroacetic acid (TFA) containing 1 mM PMSF. Automated gas phase Edman degradation was carried out for the amino terminus analysis and the amino terminus was determined to be Ala-Thr-Gln-Glu-Asp-Ala-Pro-Trp-Leu-Ala-Arg-Ile-Ser-Ser.

### Example 11

### pH-profile of proteinase TW7.

The pH-profile of proteinase TW7 at 25°C was determined using azoalbumin as a substrate. Sustrate solutions containing 0.6% azoalbumin in sodium phosphate-sodium borate buffers covering the pH range between 5.0 and 12.75 were prepared. To the azoalbumin solutions was added either 10 µl of a 1 mg/ml solution of proteinase TW7 in water or 10 µl of water (control) and the proteolytic activity of each solution was determined as described in Example 8. The pH-profile of proteinase TW7 is represented in Figure 5 wherein the percentage of maximum proteolytic activity has been plotted versus pH. The optimum pH range of proteinase TW7 has been determined to be betweena pH range of from 9 to 11.

### Example 12

### Temperature profile of proteinase TW7.

The temperature profile of proteinase TW7 in 0.05 sodium phosphate at pH 8.5 was determined by measuring the proteolytic activity over a temperature range of between 20°C and 70°C. Aliquots (1 ml) of 0.6% azoalbumin in 0.05M sodium phosphate pH 8.5 were incubated at various temperatures and after the addition of the enzyme solution (20 µl of 0.5 mg proteinase TW7 per ml of the same buffer) or 20 µl of buffer (control) the reaction was allowed to proceed for 10 minutes and then terminated upon addition of 10% trichloroacetic acid (400 µl). The temperature profile of proteinase TW7 is represented in Figure 6 wherein the percentage of maximum enzyme activity has been plotted versus temperature. As illustrated in Figure 6, the preferred temperature of proteinase TW7 ranges between 57°C to 62°C.

### Example 13

### Determination of protease stability in the presence of detergent.

The stability of protease TW7 was compared in detergent as well as non-detergent solutions with that of subtilisin Carlsberg and to that of proteinase K. Solutions of the enzymes to be evaluated were prepared in appropriate buffers so that the initial proteolytic activities of the various enzyme solutions were similar as measured by the azoalbumin assay described in Example 8. The solutions were incubated at 52°C and aliquots were drawn and measured for residual enzyme activity. The results obtained are represented in Tables 1 and 2. The residual enzyme activity is expressed as a percent of the initial enzyme activity.

**Table 1**

| Stability of protease TW7 vs. proteinase K and subtilisin Carlsberg in 0.1M sodium phosphate pH 8.0 with and without SDS. | | | | |
|---|---|---|---|---|
| Without SDS: | | | | |
| Protease | 0 Hours | 1 Hour | 2 Hours | |
| | | | | |
| Protease TW7 | 100% | 106% | 102% | |
| Proteinase K | 100% | 67% | 43% | |
| Subtilisin Carlsberg | 100% | 29% | 8% | |
| | | | | |

| With 0.5% SDS: | | | | |
|---|---|---|---|---|
| Protease | 0 Hours | 1 Hour | 2 Hours | 24 Hours |
| | | | | |
| Protease TW7 | 100% | 99% | 99% | 92% |
| Proteinase K | 100% | 32% | 9.5% | 0% |
| Subtilisin Carlsberg | 100% | 5% | 0% | 0% |

**Table 2**

| Stability of protease TW7 vs. Subtilisin Carlsberg in 0.1M sodium glycinate pH 10.30 containing 0.5% SDS | | | | |
|---|---|---|---|---|
| Protease | t = 0 | t = 1 Hr. | t = 18 Hr. | T_{½} |
| | | | | |
| Protease TW7 | 100% | 105% | 80% | 5.6 Hr. |
| Subtilisin Carlsberg | 100% | 11.3% | 0% | 0.3 Hr. |
| Subtilisin BPN | 100% | 6.6% | 0% | 0.25 Hr. |

### Example 14

### Stability of protease TW7 in detergent compositions.

The stability of protease TW7 and proteinase K were evaluated in three enzyme-containing commercial laundry detergents, Era Plus® (manufactured by Procter and Gamble), Tide® (manufactured by Procter and Gamble) and Dynamo® (manufactured by Colgate-Palmolive). The concentrated stock detergents were diluted ten times in deionized water. The endogenous protease was inactivated by incubating the diluted detergent at 65°C for 1 hour prior to addition of the protease to be evaluated. The relative activity of the protease to be evaluated in each detergent formulation was adjusted to be similar to the enzymatic activity present in the original detergent formulation.

Deactivated detergent containing the proteases to be evaluated were incubated at 52°C along with the detergent containing active endogenous enzyme. The residual proteolytic activity after different incubation periods were quantitated by withdrawing samples and assaying in accordance with the procedures as described in Example 8.

Protease TW7 was found to be stable in all detergent formulations tested. For example, in a formulation containing deactivated Era Plus®, protease TW7 retains 100% of the enzyme activity after 6 hours of incubation compared to only 12% of the endogenous enzyme. In a formulation containing deactivated Dynamo®, protease TW7 retained 76% of the enzyme activity after 29 hours of incubation.

The stability of the protease TW7 was also tested in a formulation containing the laundry detergent Wisk® which does not contain enzymes in its formulation. Following a 1:10 dilution of the stock detergent in deionized water, proteinase K or protease TW7 were added and the resulting composition was incubated at 52°C for various durations. The remaining activity was assayed using the procedures described in Example 8. Protease TW7 was more stable than proteinase K, as illustrated in Table 4, wherein after 3.5 hours of incubation, proteinase K retained only 20.1% of the original activity while protease TW7 retained approximately 90% of its original enzymatic activity.

**Table 3**

| Detergent Formulation (Enzyme) | | | |
|---|---|---|---|
| | | Time | |
| Era Plus® | | | |
| | 3 h | 6 h | |
| | | | |
| (Protease TW7) | 95 | 90 | |
| (Proteinase K) | 79 | 64 | |
| (Endogenous enzyme) | 36 | 11 | |
| | | | |

| Tide® | | | |
|---|---|---|---|
| | 1 h | 2 h | 24 h |
| | | | |
| (Protease TW7) | 107 | 104 | 64 |
| (Proteinase K) | 58 | 23 | 0 |
| | | | |

| Dynamo® | | | |
|---|---|---|---|
| | 29 h | | |
| | | | |
| (Protease TW7) | 76 | | |
| (Proteinase K) | 34.4 | | |
| (Endogenous enzyme) | 22 | | |
| | | | |

| Wisk® | | | |
|---|---|---|---|
| | 1.3 hr | 3.5 hr | |
| | | | |
| (Protease TW7) | 95.7 | 89.5 | |
| (Proteinase K) | 44.3 | 20.1 | |

Experiments were carried out as described in Example 14. Percentage of the original activity remaining after different time points are depicted.

### Example 15

### Expression of proteinase TW7.

An oligonucleotide adaptor was synthesized to insert the gene coding the mature protease TW7 in the expression vector pCFM 1156 for expression in E. coli. The adaptor has the following sequence:

AATTCTAGAAGGAGGAATAACATATGGCCACCCAGGAAGACGCCC
GATCTTCCTCCTTATTGTATACCGGTGGGTCCTTCTGCGGGTAC

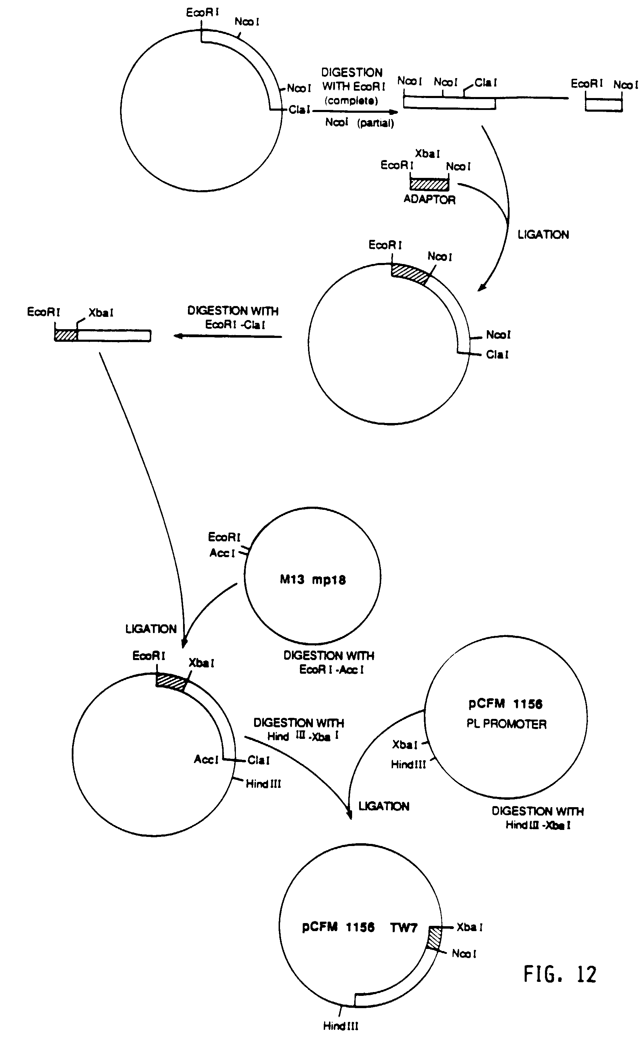

The construction of the recombinant plasmid for expression in E. coli involved the following steps:
(1) Construction of a double-stranded oligonucleotide, which contains the portion of the expression vector pCFM 1156 from the XbaI site up to the NdeI site including the ATG sequence, followed by the amino terminal sequence of the mature protease TW7 gene starting from GCC up to the first NCoI site. Additional nucleotides were added on the 5' end to obtain an extra EcoRI site that will facilitate the construction.
(2) As illustrated in the Figure 12, the cDNA for protease TW7 was digested completely with the restriction enzyme EcoRI and partially with NCoI to obtain the large EcoRI-NcoI fragment, to which kinased EcoRI-NcoI, adapter was ligated to obtain a cDNA gene for protease TW7 with the adaptor.
(3) The reconstructed cDNA for protease TW7 was then digested with EcoRI and Cla to obtain the gene for the protease TW7, which was inserted into M13mp18 which was already digested with EcoRI and AccI.
(4) The M13mp18 containing the protease TW7 gene was digested with the restriction enzymes XbaI and HindIII to rescue the protease TW7 gene.
(5) The expression vector pCFM 1156 was also digested with the restriction enzymes XbaI and HindIII to insert the protease TW7 gene obtained from M13 mp18 as a XbaI and HindIII fragment.

The resulting recombinant expression plasmid thus constructed contained a pL promoter, a Shine Dalgarno sequence, an ATG followed by the nucleotide sequence coding for the mature protease TW7 protein.

Competent E. coli FMII cells (In⁻, ptr3⁻) were transformed with this recombinant plasmid.

Plasmid DNA was isolated from the transformed cells and the positive clones were identified by digesting the plasmid DNA with XbaI and HindIII. One of the positive clones was grown overnight to inoculate a medium of Luria broth containing 20 µg kanamycin/ml. Cells were grown at 30°C up to an optical density of 0.25 at 600 nm. The temperature of incubation was then shifted to 42°C for 2 hours for the induction of plasmid born gene products.

E. coli cells were collected by low speed centrifugation. The pellet was weighed and then suspended in 10 volumes of 50 mM Tris-HCl, pH 7.5. The cells were lysed by three passes through a French press. The pellet fraction obtained after another centrifugation was extracted with 5M urea, 50 mM Tris-HCl, pH 8.0. The urea soluble proteins were analyzed using various techniques.

The proteins were reduced with β mercaptoethanol and electrophoresed in a 10% polyacrylamide gel in the presence of SDS. A unique 35,000 dalton protein was the major band present in the urea soluble fraction derived from E. coli harboring the recombinant plasmid but not the vector alone. It comigrated with the protease TW7 purified from T. album. This protein also reacted specifically with the antibody raised against the fungal protease TW7 in a Western blot analysis. This protein was isolated from polyacrylamide gel to identify the sequences in the amino terminal region, which matched with that of the fungal protease TW7.

The recombinant protease TW7 is enzymatically inactive when isolated from E. coli prior to refolding. For reactivation, the protein was suspended in 8M urea, 10 mM DTT, 25 mM Tris-HCl, pH 8.5 in a final concentration of 1 mg protein per ml and then bound to DE52 resin preequillibrated with 25 mM Tris-HCl, pH 8.5 at room temperature. Urea was removed by washing the resin with the same buffer without and then with 4 mM glutathione (reduced) and 0.4 mM glutathione (oxidized). After overnight incubation of the resins in the presence of the above reagents, i.e., 25 mM Tris-HCl, pH 8.5, 4 mM glutathione (reduced) and 0.4 mM glutathione (oxidized), the protein was eluted from the column with 50 mM Tris-HCl, pH 7.5 conaining 0.3M NaCl and precipitated with 3 volumes of acetone. This renatured protein had protease activity against the chromogenic substrate (Example 1) and casein.

### Example 16

### Isolation and purification of proteinase TW3 from the culture broth of Tritirachium album Limber.

0.5 liter of Tritirachium album Limber culture broth obtained after 15 days of fermentation in the BSA-glucose media described in Example 1 was centrifuged at 15,000 g for 10 min. Proteins in the clear supernatant were precipitated with ammonium sulfate (180 g) and collected by centrifugation. The precipitate was suspended in 0.02 M sodium phosphate pH 6.0 (50 ml) and following removal of insoluble material by centrifugation the proteins in the supernatant were reprecipitated with acetone (2.5 volumes). The precipitate was collected by centrifugation and collected on a sintered glass funnel. The precipitate was dissolved in water (40 ml) and the solution was dialyzed at 4°C against 0.02 M sodium phosphate at pH 6.0. The dialyzed solution was cleared by centrifugation and passed through a column (2.5 x 10 cm) of carboxylmethyl-cellulose (CM-52, Whatman) at a rate of 2 ml per minute, followed by washing the column with 0.02M sodium phosphate pH 6.0 (30 ml). The flow through and washing solutions were combined and the proteins were precipitated by adding 2.5 volumes of acetone.

Elution from the CM-52 column was accomplished with a linear gradient of 0 to 0.4M NaCl in sodium phosphate pH 6.0. Fractions were assayed spectrophotometrically (at 420 nm) for proteolytic activity at pH 8.2 as described in Example 8. Peak fractions containing the enzyme activity were pooled, dialyzed at 4°C against water and then lyophilized. SDS-PAGE of 2-mercaptoethanol treated sample showed protease TW3 as a major band (>96% based on Coomassie blue staining) having an apparent molecular weight of 31,000 daltons.

### Example 17

### Amino terminus analysis of protease TW3.

Protease TW3 was purified from the culture supernatant as described in Example 16. Purified protein was then inactivated with PMSF (phenylmethylsulfonylfluoride) and further purified using HPLC. The amino terminus was determined by reconstituting the protein in 50% trifluoroacetic acid (TFA) containing 1 mM PMSF. Automated gas phase Edman degradation was carried out for the amino terminus analysis. The amino terminus was found to be Ala-Glu-Gln-Arg-Asn-Ala-Pro-Trp-Gly-Leu-Ala-Arg-Ile-Ser-Ser-Thr.

### Example 18

### pH-profile of proteinase TW3.

The pH-profile of protease TW3 at 25°C was determined using azocasein as a substrate. Sustrate solutions (0.6%) M buffers covering the pH range of between 5.0 and 10 were made. To these azocasein solutions either 20 µl of an 1 mg/ml solution of protease TW3 in water or 20 µl of water (control) was added and the proteolytic activity was determined as described in Example 8. The pH-profile of proteinase TW3 is represented in Figure 10 wherein the percentage of maximum activity is plotted versus pH. The pH optimum of protease TW3 was determined to be pH 9.

### Example 19

### Temperature profile of proteinase TW3.

The temperature profile of protease TW3 in 0.05 sodium phosphate at pH 8.5 was determined from the proteolytic activity (azocasein assay) in the temperature range between 4°C and 65°C. For these measurements, aliquots (1 ml) of 0.6% azocasein in 0.05M sodium phosphate pH 8.5 were incubated at various temperatures and following the addition of the enzyme solution (20 µl of 0.5 mg proteinase TW3 per ml of the same buffer) or 20 µl of buffer alone (control) the reaction was allowed to proceed for 10 minutes and then terminated upon addition of 10% trichloroacetic acid (400 µl). The temperature profile of protease TW3 is represented in Figure 11 wherein the percentage of maximum proteolytic activity has been plotted versus temperature. As illustrated in Figure 5, the optimum temperature of protease TW3 ranges between 55°C to 60°C.

### Example 20

### Comparative Stability Study of the Protease TW3 and Subtilisin.

Protease TW3 and a commercial subtilisin (Sigma, protease VII, Cat. No. 5255) were tested for their stability at 52°C in the absence of substrates. Tests were carried out at three different pH values (4.0, 8.0 and 10.0).

Approximately 0.2 mg of enzyme/ml was incubated at 50°C. The remaining activity after a defined time interval was quantitated by withdrawing 10 µl of sample and assaying the proteolytic activity as outlined in Example 8. Table 4 represents the results obtained. The data represents the retained enzymatic activity as a percent of the original enzymatic activity. Protease TW3 demonstrated the best stability. For example, at pH 8.0, after one hour of incubation at 50°C, 90% of the original activity of protease TW3 was retained, while only 2% of the original activity of subtilisin was retained. Following 2 hours of incubation, 96% of protease TW3 activity remained, compared to 0.6% of the activity of the subtilisin. Protease TW3 was also more stable than subtilisin at pH 4.0 and 10.0.

**Table 4**

| | | Time | |
|---|---|---|---|
| pH 8.0: | | | |
| Enzyme | 1h | 2h | 3h |
| | | | |
| TW3 | 89.5 | 96.3 | 87.4 |
| Subtilisin | 2.1 | 0.6 | 0.8 |
| | | | |

| pH 10.0: | | | |
|---|---|---|---|
| Enzyme | 1h | 2h | 3h |
| | | | |
| TW3 | 96.8 | 82.7 | 74 |
| Subtilisin | 30.5 | 9.8 | 3.5 |
| | | | |

| pH 4.0: | | | |
|---|---|---|---|
| Enzyme | 1h | 2h | 3h |
| | | | |
| TW3 | 49.3 | 36 | 20.5 |
| Subtilisin | 0 | 0 | 0 |

### Example 21

### Stability of Protease TW3 in Commercial Laundry Detergents.

The stability of protease TW3, as well as of subtilisin were tested in three enzyme-containing commercial laundry detergents. These were Era Plus® (manufactured by Procter and Gamble), Tide® (manufactured by Procter and Gamble) and Dynamo® (manufactured by Colgate-Palmolive). The concentrated stock detergents were diluted 200 fold in deionized water. The endogenous protease was inactivated by incubating the diluted detergent at 65°C for 1 hour, prior to adding either protease TW3, protease TW7, proteinase K or subtilisin to achieve the enzyme activity present in the original detergent formulation.

Inactivated detergent with added proteases was incubated at 50°C for various time points along with the detergent containing active endogenous enzyme. The residual proteolytic activity remaining after different incubation periods was quantitated by withdrawing samples and assaying in accordance with the procedures described in Example 8.

As illustrated in Table 5, protease TW3 is very stable in all detergent formulations tested. For example, in a formulation containing Era Plus®, 94% of its activity was retained after 1 hour of incubation while the formulation containing endogenous enzyme was completely inactivated. In a formulation containing Dynamo, 80% of the activity of protease TW3 remained after one hour of incubation, compared to only a nondetectable level of activity in a formulation containing endogenous enzyme. In a formulation containing Tide®, 48% of the original activity of TW3 was retained, while 23% of the activity in the formulation containing endogenous enzyme remained after one hour. In all instances subtilisin was inactivated within one hour.

The stability of the protease TW3 was also tested in the laundry detergent Wisk® which does not contain enzyme in its formulation. Following a 1:200 dilution of the stock detergent in deionized water, subtilisin or protease TW3 were added to the diluted detergent and the samples were incubated at 50°C. The proteolytic activity was assayed using the procedure outlined in Example 8. The results obtained are represented in Table 5.

**Table 5**

| Detergent Formulation (Enzyme) | | | | |
|---|---|---|---|---|
| | | Time | | |
| | 10' | 20' | 30' | 60' |
| ERA Plus® | | | | |
| | | | | |
| (TW7) | 83.4 | 94.5 | 87.7 | 89 |
| (TW3) | 87.7 | 89.1 | 92.2 | 94.8 |
| (Subtilisin) | 3.9 | 1.3 | 1.5 | 1.8 |
| (Endogenous enzyme) | 50.0 | 37.7 | 24.7 | 0 |
| (Proteinase K) | 83.2 | 84.1 | 86.7 | 79.7 |
| | | | | |

| Tide® | | | | |
|---|---|---|---|---|
| | | | | |
| (TW7) | 103.5 | 111 | 99.6 | 110 |
| (TW3) | 78 | 55.7 | 68 | 48 |
| (Subtilisin) | 1.8 | 0.8 | 0.74 | 0.5 |
| (Endogenous enzyme) | 22.6 | 22 | 21.9 | 23.5 |
| (Proteinase K) | 83 | 75 | 62 | 35.4 |
| | | | | |

| Dynamo® | | | | |
|---|---|---|---|---|
| | | | | |
| (TW7) | 84.9 | 90 | 85.9 | 89.3 |
| (TW3) | 83 | 90.8 | 82 | 87.5 |
| (Subtilisin) | 30 | 10.8 | 3.6 | 0.5 |
| (Endogenous enzyme) | 52 | 32.5 | 15.1 | 0 |
| (Proteinase K) | 81.3 | 83 | 79.9 | 87.4 |
| | | | | |

| Wisk® | | | | |
|---|---|---|---|---|
| | | | | |
| (TW7) | 89.1 | 87.1 | 90.8 | 90 |
| (TW3) | 58.3 | 43.5 | 28.2 | 6 |
| (Subtilisin) | 21 | 36 | 0.7 | 0.5 |
| (Endogenous enzyme) | 0 | 0 | 0 | 0 |
| (Proteinase K) | 57.6 | 39.7 | 19.7 | 2.8 |

Various other examples and modifications of the foregoing description and examples will be apparent to a person skilled in the art after reading the present disclosure without departing from the spirit and scope of the invention, and it is intended that all such examples or modifications be included within the scope of the appended claims.

## Claims

1. A serine protease having an amino acid sequence as set forth in Figure 3.

2. A serine protease having an amino acid sequence as set forth in Figure 8.

3. A serine protease according to Claim 1 or 2 having an amino terminal residue at Ala¹.

4. A DNA sequence for use in securing expression in a procaryotic or eucaryotic host cell of a serine protease, said sequence selected from
a) the DNA sequences set forth in Figure 1, 2 or 7; and
b) DNA sequences which hybridize to the sequences defined in (a) and code for the protease encoded by the sequences in (a).

5. A composition comprising an effective amount of a serine protease according to Claim 1, 2 or 3 in a detergent composition.

6. An expression vector capable, in a transformed cell culture, of expressing a DNA sequence according to Claim 4.

7. A cell culture transformed with an expression vehicle according to Claim 6.

8. A microorganism according to Claim 7 obtained by transforming an *E. coli* strain.

9. An *E. coli* strain capable of producing a serine protease having an amino acid sequence set forth in Figure 3.

10. An *E. coli* strain capable of producing a serine protease having an amino acid sequence set forth in Figure 8.

11. A serine protease having an amino acid sequence as set forth in Figure 3 or Figure 8 and characterized by being the product of procaryotic or eucaryotic expression of an exogenous DNA sequence.

12. A serine protease according to Claim 11, wherein the exogenous DNA sequence is a cDNA sequence.

13. A serine protease according to Claim 11, wherein the exogenous DNA sequence is a genomic DNA sequence.

14. A serine protease according to Claim 11, wherein the exogenous DNA sequence is a manufactured DNA sequence.

15. A serine protease according to Claim 11, which is the product of *E. coli* expression.

16. A process for the production of a serine protease, which process comprises the steps of:
a) growing under suitable nutrient conditions procaryotic or eucaryotic host cells transformed or transfected with a DNA vector including a DNA sequence according to Claim 4 useful in securing expression in a procaryotic or eucaryotic host cell of a desired serine protease; and
b) isolating the desired serine protease produced by the expression of said DNA sequences in said host cell.

## Patentansprüche

1. Eine Serinprotease mit einer Aminosäuresequenz, wie sie in Figur 3 angegeben ist.

2. Eine Serinprotease mit einer Aminosäuresequenz, wie sie in Figur 8 angegeben ist.

3. Eine Serinprotease nach Anspruch 1 oder 2, mit einem aminoterminalen Rest an Ala¹.

4. Eine DNA-Sequenz zur Verwendung für die Sicherstellung der Expression einer Serinprotease in einer prokaryontischen oder eukaryontischen Wirtszelle, wobei besagte Sequenz ausgewählt ist aus
a) den DNA-Sequenzen, die angegeben sind in Figur 1, 2 oder 7; und
b) DNA-Sequenzen, die an die in (a) definierten Sequenzen hybridisieren und für die durch die Sequenzen in (a) codierte Protease codieren.

5. Eine Zusammensetzung, die eine wirksame Menge einer Serinprotease nach Anspruch 1, 2 oder 3 umfaßt, in einer Reinigungsmittelzusammensetzung.

6. Ein Expressionsvektor, der in der Lage ist, in einer transformierten Zellkultur, eine DNA-Sequenz nach Anspruch 4 zu exprimieren.

7. Eine Zellkultur, die mit einem Expressionsvehikel nach Anspruch 6 transformiert ist.

8. Ein Mikroorganismus nach Anspruch 7, der durch Transformation eines E. coli-Stammes erhalten ist.

9. Ein E. coli-Stamm, der in der Lage ist, eine Serinprotease mit einer Aminosäuresequenz, die in Figur 3 angegeben ist, zu produzieren.

10. Ein E. coli-Stamm, der in der Lage ist, eine Serinprotease mit einer Aminosäuresequenz, die in Figur 8 angegeben ist, zu produzieren.

11. Eine Serinprotease mit einer Aminosäuresequenz, wie sie in Figur 3 oder Figur 8 angegeben ist, und dadurch gekennzeichnet, daß sie das Produkt prokaryontischer oder eukaryontischer Expression einer exogenen DNA-Sequenz ist.

12. Eine Serinprotease nach Anspruch 11, wobei die exogene DNA-Sequenz eine cDNA-Sequenz ist.

13. Eine Serinprotease nach Anspruch 11, wobei die exogene DNA-Sequenz eine genomische DNA-Sequenz ist.

14. Eine Serinprotease nach Anspruch 11, wobei die exogene DNA-Sequenz eine künstlich hergestellte DNA-Sequenz ist.

15. Eine Serinprotease nach Anspruch 11, die das Produkt von E. coli-Expression ist.

16. Ein Verfahren zur Herstellung einer Serinprotease, wobei das Verfahren die Schritte umfaßt:
a) Züchten prokaryontischer oder eukaryontischer Wirtszellen, die mit einem DNA-Vektor, einschließlich einer DNA-Sequenz nach Anspruch 4, der für die Sicherstellung der Expression einer gewünschten Serinprotease in einer prokaryontischen oder eukaryontischen Wirtszelle brauchbar ist, transformiert oder transfiziert worden sind, unter geeigneten Nährstoffbedingungen; und
b) Isolieren der gewünschten Serinprotease, die durch die Expression besagter DNA-Sequenzen in besagter Wirtszelle produziert worden ist.

## Revendications

1. Protéase de sérine ayant une séquence aminoacide comme indiqué sur la figure 3.

2. Protéase de sérine ayant une séquence aminoacide comme indiqué sur la figure 8.

3. Protéase de sérine selon la revendication 1 ou 2 présentant un résidu terminal aminé à Ala¹.

4. Séquence d'ADN pour une utilisation en associant une expression dans une cellule hôte procaryote ou eucaryote d'une protéase de sérine, ladite séquence est choisie à partir :
a) des séquences d'ADN indiquées sur la figure 1, 2 ou 7 ; et
b) de séquences d'ADN qui s'hybrident aux séquences définies dans (a) et codent pour la protéase codée par les séquences dans (a).

5. Composition comprenant une quantité efficace d'une protéase de sérine selon la revendication 1, 2 ou 3 dans une composition détergente.

6. Vecteur d'expression capable, dans une culture de cellules transformées, d'exprimer une séquence d'ADN selon la revendication 4.

7. Culture cellulaire transformée avec un véhicule d'expression selon la revondication 6.

8. Micro-organisme selon la revendication 7, obtenu en transformant une souche E. coli.

9. Souche E. coli capable de produire une protéase de sérine ayant une séquence aminoacide indiquée sur la figure 3.

10. Souche E. coli capable de produire une protéase de sérine ayant une séquence aminoacide indiquée sur la figure 8.

11. Protéase de sérine présentant une séquence aminoacide, comme indiqué sur la figure 3 ou la figure 8, et caractérisée en étant le produit d'expression procaryote ou eucaryote d'une séquence d'ADN exogène.

12. Protéase de sérine selon la revendication 11, dans laquelle la séquence d'ADN exogène est une séquence d'ADNc.

13. Protéase de sérine selon la revendication 11, dans laquelle la séquence d'ADN exogène est une séquence d'ADN génomique.

14. Protéase de sérine selon la revendication 11, dans laquelle la séquence d'ADN exogène est une séquence d'ADN fabriquée.

15. Protéase de sérine selon la revendication 11, qui est le produit de l'expression E. coli.

16. Procédé pour la production d'une protéase de sérine, lequel procédé comprend les étapes de :
a) faire croître, sous des conditions nutritives appropriées, des cellules hôtes procaryotes ou eucaryotes, transformées ou transfectées avec un vecteur d'ADN incluant une séquence d'ADN selon la revendication 4, utile en associant l'expression dans une cellule hôte procaryote ou eucaryote d'une protéase de sérine souhaitée ; et
b) isoler la protéase de sérine souhaitée, produite par l'expression desdites séquences d'ADN, dans ladite cellule hôte.
